Europäisches Patentamt

European Patent Office

Office européen des brevets

(11) Veröffentlichungsnummer: **0 229 998 B1**

## (12) EUROPÄISCHE PATENTSCHRIFT

(45) Veröffentlichungstag der Patentschrift: **15.07.92**

(21) Anmeldenummer: **86117481.1**

(22) Anmeldetag: **16.12.86**

(51) Int. Cl.⁵: **C12N 15/26**, C12P 21/02, C07K 15/00, C12N 1/21

(54) **Fusionsproteine mit eukaryotischem Ballastanteil.**

(30) Priorität: **21.12.85 DE 3545565**
**30.10.86 DE 3636903**

(43) Veröffentlichungstag der Anmeldung:
**29.07.87 Patentblatt 87/31**

(45) Bekanntmachung des Hinweises auf die
Patenterteilung:
**15.07.92 Patentblatt 92/29**

(84) Benannte Vertragsstaaten:
**AT BE CH DE ES FR GB GR IT LI LU NL SE**

(56) Entgegenhaltungen:
**EP-A- 0 155 655**
**EP-A- 0 158 198**
**EP-A- 0 161 937**

(73) Patentinhaber: **HOECHST AKTIENGESELL-
SCHAFT
Postfach 80 03 20
W-6230 Frankfurt am Main 80(DE)**

(72) Erfinder: **Habermann, Paul, Dr.
Heimchenweg 80
W-6230 Frankfurt am Main 80(DE)**

EP 0 229 998 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es wurden bereits Fusionsproteine vorgeschlagen, die durch einen C- oder N-terminalen Anteil gekennzeichnet sind, der im wesentlichen den ersten 100 Aminosäuren von Interleukin-2 entspricht (deutsche Patentanmeldung P 35 41 856.7). entsprechend der europäischen Anmeldung 86116140.4 = EP-A2-0 227 938 Der Interleukin-2-Anteil kann sich hierbei von Säuger-Interleukin-2 ableiten, beispielsweise vom Mäuse- oder Ratten-Interleukin-2, die aus der europäischen Patentanmeldung mit der Veröffentlichungsnummer (im folgenden "EP-A") 0 091 539 bekannt sind, bevorzugt von Human-Interleukin-2. Diese Fusionsproteine sind in der Wirtszelle überraschend stabil und können aufgrund ihrer geringen Löslichkeit einfach von den löslichen wirtseigenen Proteinen abgetrennt werden.

In Weiterentwicklung dieses Erfindungsgedankens wurde nun überraschenderweise gefunden, daß auch wesentlich kleinere Anteile des Interleukin-2-Moleküls als "Ballast"-Anteil für derartige Fusionsproteine geeignet sind. Die Erfindung betrifft demzufolge Fusionsproteine mit einem Anteil von Interleukin-2 (IL-2) und einem gewünschten Protein, gekennzeichnet durch einen C- oder N-terminalen Anteil, der im wesentlichen der Aminosäurefolge von IL-2 entspricht, der aber biologisch nicht aktiv ist, wobei Fusionsproteine ausgenommen sind, deren IL-2-Anteil im wesentlichen mindestens den ersten 100 Aminosäuren von IL-2 entspricht. Weitere Aspekte der Erfindung sind in den Patentansprüchen definiert. Bevorzugte Ausgestaltungen werden im folgenden näher erläutert.

Besonders vorteilhaft geht man von dem synthetischen Gen für Human-Interleukin-2 (im folgenden "IL-2") aus, das in der EP-A 0 163 249 beschrieben und im Anhang wiedergegeben ist. Dieses synthetische Gen enthält eine Reihe von singulären Restriktionsschnittstellen, die es erlauben, die für IL-2 kodierende DNA in "handliche" Segmente zu zerlegen. Mit diesen Segmenten kann nach dem Baukastenprinzip der Ballastanteil für Fusionsproteine maßgeschneidert werden, wobei man je nach Kombination der Segmente und nach der Art des gewünschten Proteins leicht- bis schwerlösliche Fusionsproteine erhält.

Die Erfindung gestattet es also, je nach der möglichen oder gewünschten Aufarbeitung des Produktes die vorteilhafteste Löslichkeit anzusteuern, also eine hohe Löslichkeit, wenn das Produkt chromatographisch gereinigt werden soll, beispielsweise mit Hilfe einer Antikörpersäule, oder aber eine geringe Löslichkeit, wenn zur Vorreinigung die wirtseigenen löslichen Proteine abgetrennt, beispielsweise abzentrifugiert werden sollen.

Ein besonderer Vorteil der Erfindung liegt darin, daß man Fusionsproteine mit einem sehr kleinen "Ballastanteil" herstellen kann, da hierdurch die relative Ausbeute am gewünschten Protein beträchtlich erhöht wird.

Ein weiterer Vorzug der Erfindung liegt darin, daß der "Ballastanteil" so konstruiert werden kann, daß er die räumliche Struktur des gewünschten Proteins möglichst wenig behindert und so beispielsweise eine Auffaltung nicht verhindert.

Bei der Spaltung der Fusionsproteine erhält man neben dem gewünschten Protein den "Ballastanteil", also das IL-2-Derivat. Dieses kann IL-2-Aktivität aufweisen (T-Zell-Proliferationstest) oder an IL-2-Rezeptoren binden. Das erfindungsgemäße "Baukastenprinzip" kann also auch dazu dienen, als "Nebenprodukte" IL-2-Derivate zu erzeugen, die die biologischen Aktivitäten des IL-2 mehr oder weniger ausgeprägt zeigen.

Besonders zweckmäßige Ausgestaltungen der Erfindung werden im folgenden anhand des synthetischen Gens erläutert, das in der EP-A 0 163 249 beschrieben ist. Dieses Gen ist am 5'-Ende mit der Restriktionsendonuklease EcoRI und am 3'-Ende mit SalI geschnitten. Außer den drei singulären Restriktionsschnittstellen für die Enzyme PstI, XbaI und SacI, die zur Konstruktion dieses Gens dienten, sind auch die singulären Schnittstellen für MluI und PvuI günstig angeordnet. Bezeichnet man die zwischen diesen Schnittstellen liegenden Sequenzen mit A bis F, so kann man das synthetische Gen wie folgt systematisch darstellen

(EcoRI)-A-PstI-B-MluI-C-XbaI-D-SacI-E-PvuI-F-(SalI)

Die Segmente A bis F stellen somit besonders geeignete "Bausteine" für das erfindungsgemäße Baukastensystem dar. In dieser Darstellung entspricht also der "Ballastanteil" für die Fusionproteine entsprechend der deutschen Patentanmeldung P 35 41 856.7 den Segmenten A bis E und für das in der gleichen Anmeldung genannte bifunktionelle Protein mit dem gesamten IL-2-Gen allen Segmenten A bis F. Demgegenüber betreffen die erfindungsgemäßen Genkonstruktionen andere Kombinationen der Segmente A bis F, vorzugsweise mit weniger als 4 dieser Segmente, wobei das Segment A den N-Terminus der Fusionsproteins kodiert. Die Anordnung der weiteren Segmente ist beliebig, wobei gegebenenfalls entsprechende Adaptoren oder Linker eingesetzt werden. Entsprechende Adaptoren- oder Linkersequenzen können auch am C-Terminus des "Ballastanteils" eingeführt werden, die in diesem Falle auch für Aminosäuren oder kurze Aminosäuresequenzen kodieren können, die die enzymatische oder chemische Abspaltung des "Ballastanteils" vom gewünschten Protein ermöglichen oder erleichtern. Die Adapter- oder Linkersequenzen können selbstverständlich auch dazu dienen, den "Ballastanteil" für ein bestimmtes Fusionsprotein maßzuschneidern, beispielsweise zur Erzielung einer gewünschten Löslichkeit. Überraschenderweise hat es sich nämlich gezeigt, daß die Löslichkeit der Fusionsproteine nicht von der Molekülgröße abhängig ist, sondern daß vielmehr auch relativ kleine Moleküle eine geringe Löslichkeit aufweisen können. In Kenntnis dieser Zusammenhänge, die in den Beispielen im einzelnen erläutert sind, kann somit der Fachmann ohne großen experimentellen Aufwand erfindungsgemäß Fusionsproteine mit kleinem "Ballastanteil" gewinnen, die bestimmte gewünschte Eigenschaften aufweisen.

Wenn das gewünschte Protein ein eukaryotisches Protein ist, werden also erfindungsgemäß Fusionsproteine erhalten, die ausschließlich oder praktisch ausschließlich aus eukaryotischen Proteinsequenzen bestehen. Überraschenderweise werden diese Fusionsproteine jedoch von prokaryotischen Wirtszellen nicht als Fremdproteine erkannt und nicht durch wirtseigene Proteasen rasch abgebaut. Dieser Abbau erfolgt besonders häufig bei von cDNA-Sequenzen kodierten wirtsfremden Proteinen, die in Bakterien exprimiert werden sollen. Es hat sich nun gezeigt, daß cDNA-Sequenzen sehr effektiv exprimiert werden können, wenn sie in die erfindungsgemäßen Segmente "eingebettet" werden. Hierfür lassen sich spezielle Vektoren konstruieren, die zwischen den erfindungsgemäßen Sequenzen eine Polylinker-Sequenz enthalten, die mehrere Klonierungsstellen für die cDNA-Sequenzen aufweist. Sofern die einklonierte cDNA kein Stop-Codon enthält, so wird die von der cDNA-Sequenz kodierte Polypeptidsequenz durch das Polypeptid zusätzlich geschützt, für das das C-terminale Segment kodiert.

Die Spaltung des Fusionsproteins kann in an sich bekannter Weise chemisch oder enzymatisch erfolgen. Die Wahl der geeigneten Methode richtet sich vor allem nach der Aminosäuresequenz des gewünschten Proteins. Wenn dieses beispielsweise kein Methionin enthält, kann das Bindeglied Met bedeuten, worauf eine chemische Spaltung mit Chlor- oder Bromcyan erfolgt. Steht im Bindeglied am Carboxyterminus Cystein oder steht das Bindeglied für Cys, so kann eine enzymatische Cystein-spezifische Spaltung oder eine chemische Spaltung, beispielsweise nach spezifischer S-Cyanylierung, folgen. Steht im Brückenglied am Carboxyterminus Tryptophan oder das Bindeglied für Trp, so kann eine chemische Spaltung mit N-Bromsuccinimid erfolgen.

Gewünschte Proteine, die in ihrer Aminosäuresequenz nicht Asp - Pro enthalten und hinreichend säurestabil sind, können als Fusionsproteine mit diesem Brückenglied in an sich bekannter Weise proteolytisch gespalten werden. Hierdurch erhält man Proteine, die N-terminal Prolin bzw. C-terminal Asparaginsäure enthalten. Auf diese Weise können also auch modifizierte Proteine synthetisiert werden.

Die Asp-Pro-Bindung kann noch säurelabiler gestaltet werden, wenn dieses Brückenglied $(Asp)_n$-Pro bzw. Glu-$(Asp)_n$-Pro ist, wobei n 1 bis 3 bedeutet.

Beispiele für enzymatische Spaltungen sind ebenfalls bekannt, wobei auch modifizierte Enzyme mit verbesserter Spezifität eingesetzt werden können (vgl. C.S. Craik et al., Science 228 (1985) 291-297). Ist das gewünschte eukaryotische Peptid Proinsulin, so wählt man zweckmäßig eine Peptidsequenz, bei der eine durch Trypsin abspaltbare Aminosäure (Arg, Lys) an die N-terminale Aminosäure (Phe) des Proinsulins gebunden ist, beispielsweise Ala-Ser-Met-Thr-Arg, da dann die Arginin-spezifische Spaltung mit der Protease Trypsin erfolgen kann.

Enthält das gewünschte Protein nicht die Aminosäurefolge
Ile-Glu-Gly-Arg,
so kann das Fusionsprotein mit dem entsprechenden Brückenglied mit Faktor Xa gespalten werden (EP-A 0 025 190 und 0 161 973).

Das Fusionsprotein wird durch Expression in einem geeigneten Expressionssystem in an sich bekannter Weise gewonnen. Hierfür eignen sich alle bekannten Wirts-Vektor-Systeme, also beispielsweise Säugerzellen und Mikroorganismen, beispielsweise Hefen und vorzugsweise Bakterien, insbesondere E. coli.

Die DNA-Sequenz, die für das gewünschte Protein codiert, wird in bekannter Weise in einen Vektor eingebaut, der in dem gewählten Expressionssystem eine gute Expression gewährleistet.

In bakteriellen Wirten wählt man zweckmäßig den Promotor und Operator aus der Gruppe lac, tac, trp, $P_L$ oder $P_R$ des Phagen λ, hsp, omp oder einen synthetischen Promotor, wie sie beispielsweise in der deutschen Offenlegungsschrift 34 30 683 (EP-A 0 173 149) vorgeschlagen sind. Vorteilhaft ist die tac Promotor-Operator-Sequenz, die inzwischen handelsüblich ist (z.B. Expressionsvektor pKK223-3, Pharmacia, "Molecular Biologicals, Chemicals and Equipment for Molecular Biology", 1984, s. 63).

Bei der Expression des erfindungsgemäßen Fusionsproteins kann es sich als zweckmäßig erweisen, einzelne Tripletts der ersten Aminosäuren nach dem ATG-Start-Codon zu verändern, um eine eventuelle Basenpaarung auf der Ebene der mRNA zu verhindern. Solche Veränderungen, ebenso wie Veränderungen, Deletionen oder Additionen einzelner Aminosäuren im IL-2-Proteinanteil, sind dem Fachmann geläufig und ebenfalls Gegenstand der Erfindung. Beispielhaft kann eine Eliminierung von Cystein oder der Ersatz von

EP 0 229 998 B1

Cystein durch andere Aminosäuren erwähnt werden, um eine Bildung unerwünschter Disulfidbrücken zu verhindern, wie es beispielsweise aus der EP-A 109 748 bekannt ist.

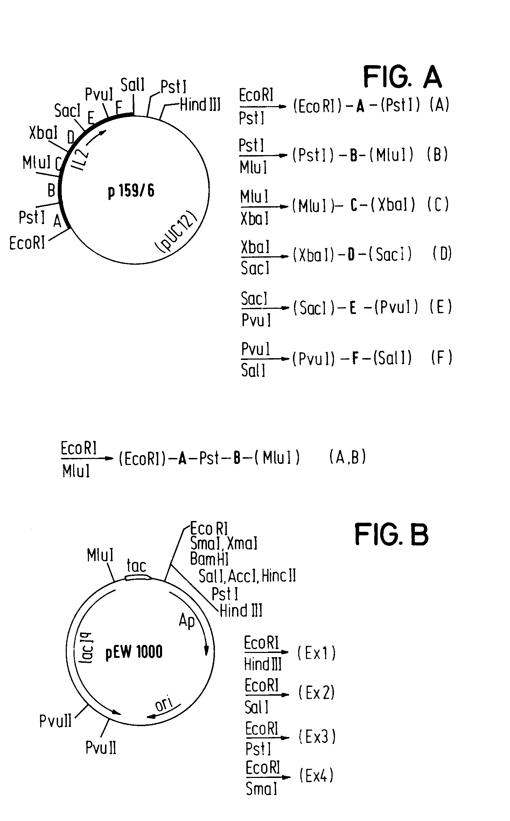

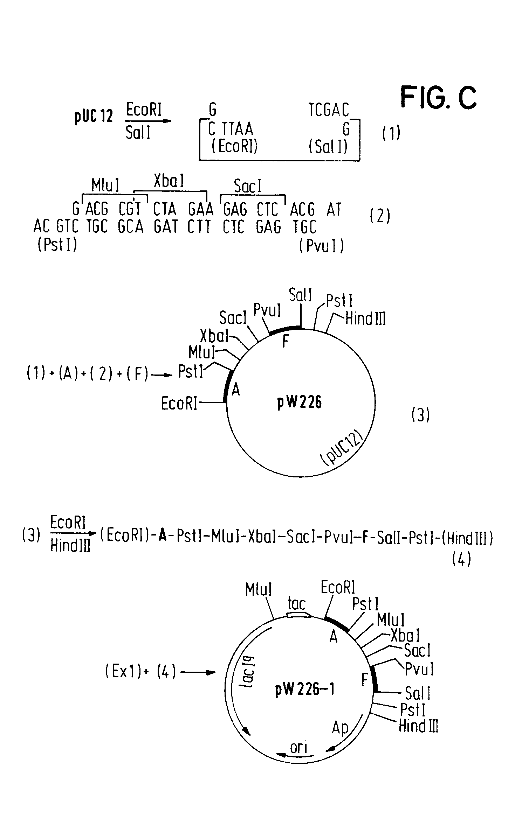

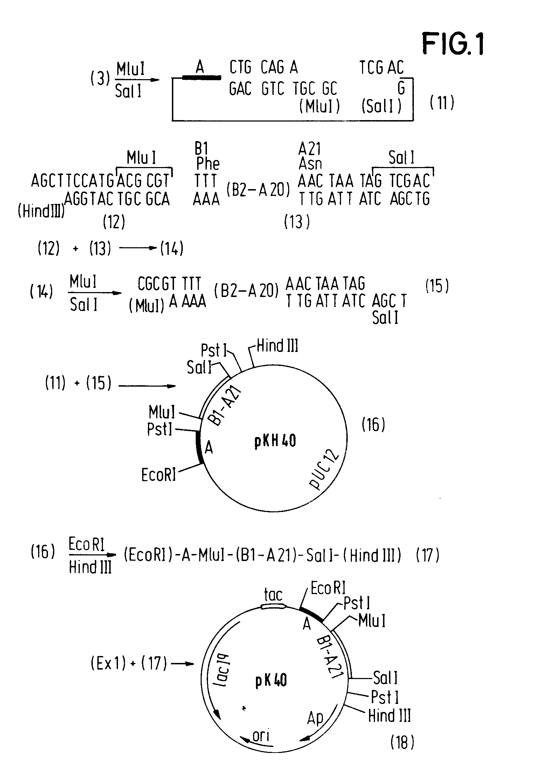

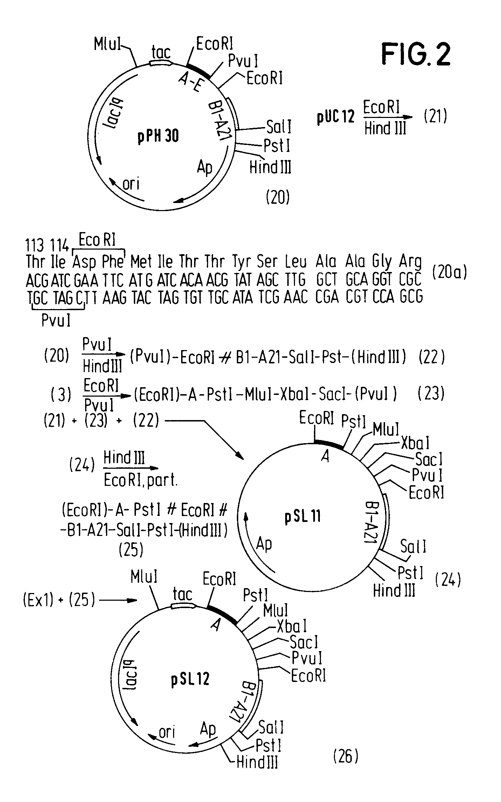

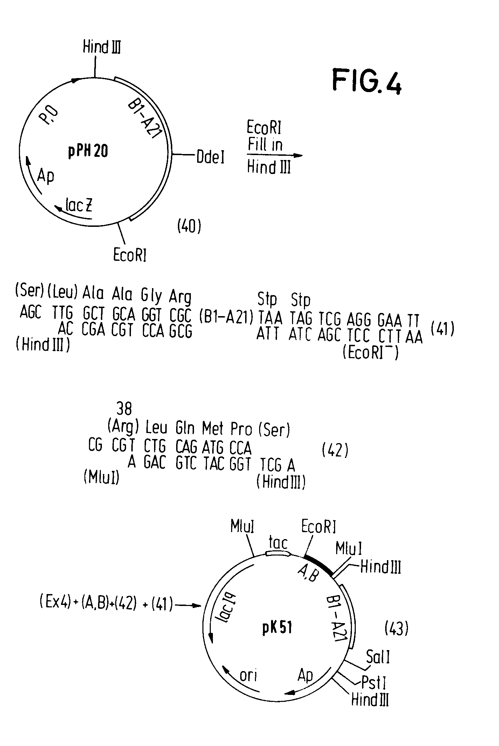

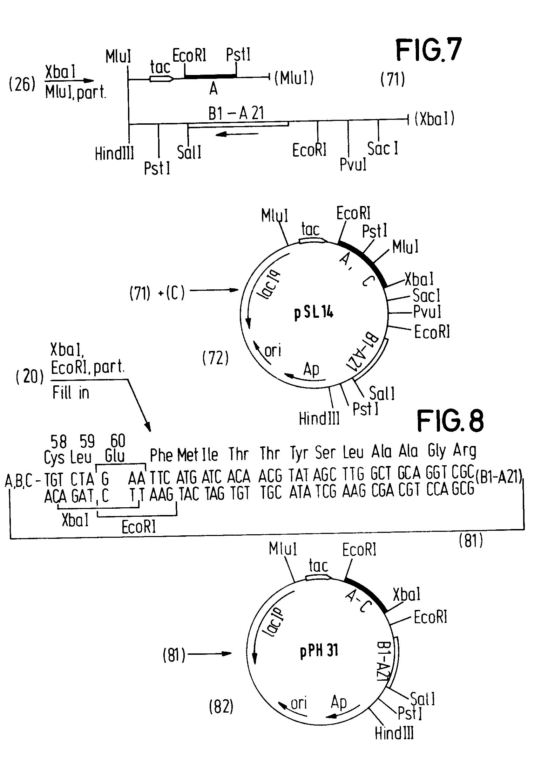

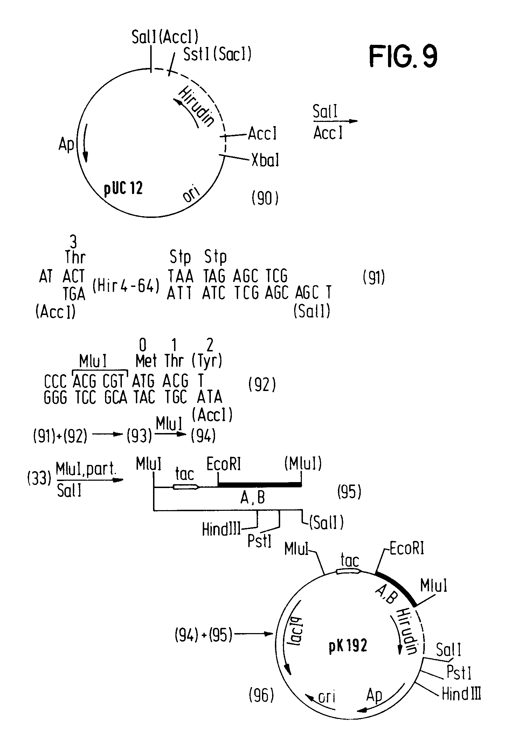

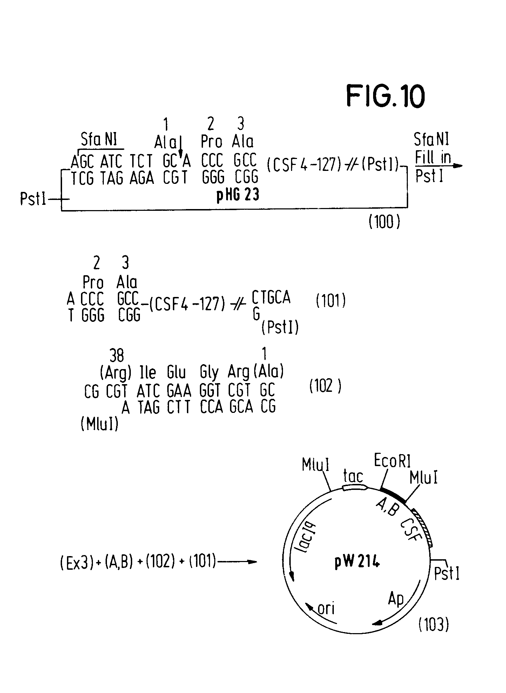

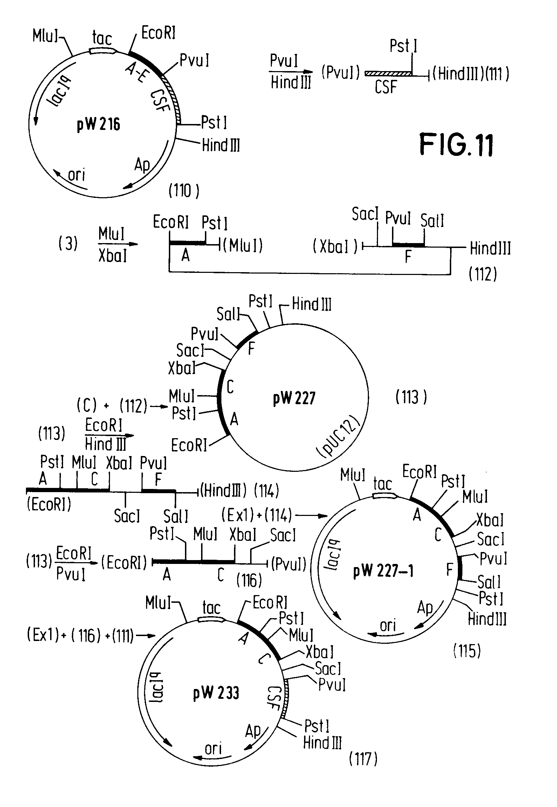

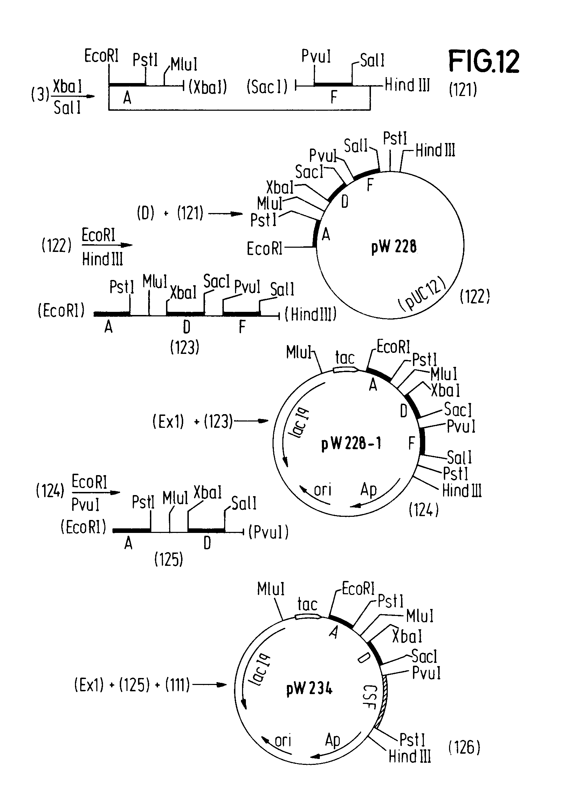

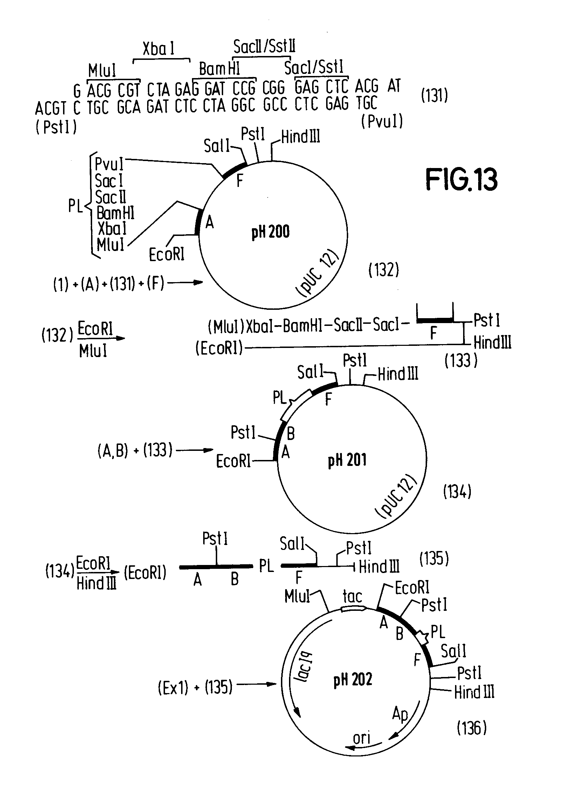

Die Figuren 1 bis 13 veranschaulichen nach Art eines Fließbildes die Verfahren der in den Beispielen gleicher Nummer beschriebenen Synthesen. Zur Erleichterung der Übersicht wurde die Herstellung der Ausgangsmaterialien bzw. Zwischenprodukte in den Figuren A bis C dargestellt. Zur Verbesserung der Übersichtlichkeit wurden in den Figuren 1 bis 13 die Bezugsziffern mit einer neuen 10er-Reihe begonnen, also in Figur 1 mit (11). Bezugsziffern von Ausgangsmaterialien, die nicht Gegenstand der vorliegenden Anmeldung sind, enden auf 0, also beispielsweise in Figur 2 (20). Die Figuren sind nicht maßstäblich gezeichnet, insbesondere im Bereich der Polylinkersequenzen ist der Maßstab entsprechend gedehnt. IL-2-Sequenzen werden durch dicke Linien charakterisiert, Strukturgene für gewünschte Proteine sind anderweitig hervorgehoben.

Die Figur A gibt eine Übersicht über die erfindungsgemäßen Segmente A bis F bzw. die Segment-Kombination A und B. Ausgangsmaterial ist das Plasmid p159/6, dessen Herstellung in der EP-A 0 163 249 eingehend beschrieben ist und durch die Figur 5 in dieser Druckschrift charakterisiert ist.

Die Figur B zeigt das Expressionsplasmid pEW1000, dessen Herstellung in der deutschen Patentanmeldung P 35 41 856.7 beschrieben und in deren Figur 1 dargestellt ist. Durch entsprechende Doppelverdauung wird dieses Plasmid in der Polylinkersequenz geöffnet, wobei die linearisierten Plasmide (Ex1) bis (Ex4) erhalten werden.

Die Figur C zeigt die Herstellung des pUC12-Derivats pW226 und des Expressionsplasmids pW226-1, die beide die Segmente A und F, getrennt durch eine Polylinkersequenz, enthalten.

Die Figur 1 zeigt die Herstellung des pUC12-Derivats pKH40 und des Expressionsplasmids pK40, die für Fusionsproteine kodieren, in denen auf die Proteinsequenz entsprechend dem Segment A, also die ersten 22 Aminosäuren von IL-2, das Brückenglied Thr-Arg folgt, worauf sich die Aminosäuresequenz des Proinsulins anschließt.

Die Figur 2 zeigt die Konstruktion des Plasmids pSL11 und des Expressionsplasmids pSL12, die für Polypeptide kodieren, in denen auf das Segment A ein Brückenglied entsprechend den Polylinkersequenzen (2) und (20a) folgt, worauf sich die Aminosäurefolge von Proinsulin anschließt.

Die Figur 3 zeigt die Konstruktion des Expressionsplasmids pK50, das für ein Polypeptid kodiert, bei dem auf die Segmente A und B, also auf die ersten 38 Aminosäuren von IL-2, unmittelbar die Aminosäuresequenz von Proinsulin folgt.

Die Figur 4 zeigt die Konstruktion des Expressionsplasmids pK51, das für ein Polypeptid kodiert, bei dem auf die Segmente A und B ein Brückenglied entsprechend den Sequenzen (42) und (41) folgt, an das sich die Aminosäuresequenz des Proinsulins anschließt.

Die Figur 5 zeigt die Konstruktion des Expressionsplasmids pK52, das sich von pK51 durch den eingeschobenen MluI-Linker (51) unterscheidet, der für die Aminosäuresequenz kodiert, die eine Spaltung mit dem Faktor Xa ermöglicht. pK52 kann auch aus pK50 (Figur 3) durch Spalten mit MluI und Einfügen des genannten MluI-Linkers gewonnen werden.

Die Figur 6 zeigt die Konstruktion des Expressionsplasmids pK53 aus pK51 (Figur 4), ebenfalls durch Einfügen des MluI-Linkers.

Die Figur 7 zeigt die Konstruktion des Expressionsplasmids pSL14 aus pSL12 (Figur 2) durch Einfügen des Fragments C in den Polylinker. Hierdurch wird das Segment C unmittelbar an das Segment A angefügt. Im folgenden Polylinker entsprechen die ersten beiden Aminosäuren (jeweils Glu) den Aminosäuren 60 und 61 von IL-2. Somit besteht also der Il-2-Anteil aus den Aminosäuren 1 bis 22 und 37 bis 61. Die weitere Aminosäurefolge entspricht derjenigen, für die das Plasmid pSL12 (Figur 2) kodiert.

Die Figur 8 zeigt die Konstruktion des Expressionsplasmids pPH31, das für ein Fusionsprotein kodiert, bei dem auf die Segmente A bis C ein Brückenglied folgt, das in der Sequenz (81) dargestellt ist, woran sich die Aminosäurefolge von Proinsulin anschließt.

Die Figur 9 zeigt die Konstruktion des Plasmids pK192, das für ein Fusionsprotein kodiert, in dem auf die Segmente A und B Methionin und hierauf die Aminosäurefolge von Hirudin folgt.

Die Figur 10 zeigt die Konstruktion des Plasmids pW214, das für ein Fusionsprotein kodiert, in dem auf die Segmente A und B die Aminosäuresequenz folgt, die eine Spaltung mit Faktor Xa ermöglicht, woran sich die Aminosäuresequenz des Granulocyten-Makrophagen "Colony Stimulating Factor" (CSF) anschließt.

Die Figur 11 zeigt die Konstruktion des Expressionsplamids pW233, das für ein Fusionsprotein kodiert, in dem auf die Segmente A und C (entsprechend den Aminosäuren 1 bis 22 und 37 bis 61 von IL-2) das Brückenglied Leu-Thr-Ile-Asp-Asp-Pro folgt, worauf sich die Aminosäuresequenz von CSF anschließt.

Die Figur 12 zeigt die Konstruktion des Expressionsplasmids pW234, das für ein Fusionsprotein mit der folgenden Aminosäuresequenz kodiert: Auf das Segment A (Aminosäuren 1 bis 22) folgt ein Brückenglied Thr-Arg, hierauf das Segment D (Aminosäuren 59 bis 96 von IL-2), als weiteres Bindeglied Thr-Asp-Asp-Pro

4

und schließlich CSF.

Die Figur 13 zeigt die Konstruktion des Plasmide pH200 und pH201 sowie des Expressionsplasmids pH202. Diese Plasmide haben einen Polylinker zwischen den Segmenten A und F bzw. A, B und F angeordnet, in deren zahlreiche Schnittstellen Fremd-DNA kloniert werden kann. Diese Plasmide eignen sich besonders zur Klonierung von cDNA-Sequenzen.

In den folgenden Beispielen, deren Numerierung mit der der Figuren übereinstimmt, wird die Erfindung näher erläutert. Sofern keine anderen Angaben gemacht sind, beziehen sich Prozentangaben auf das Gewicht.

Beispiel A

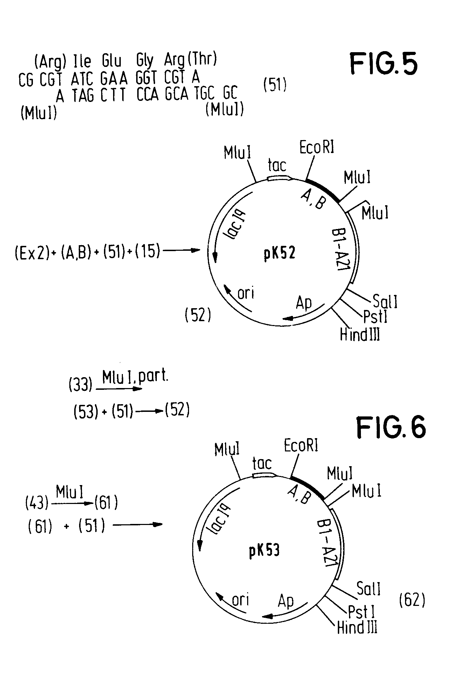

Das Ausgangsplasmid p159/6 ist in der EP-A 0 163 249 beschrieben (Figur 5). Die dort als "IL-2" bzw. im Text als "DNA-Sequenz I" definierte Sequenz ist in der Figur A in die Segmente A bis F aufgeteilt, die durch die Schnittstellen für die Enzyme EcoRI, PstI, MluI, XbaI, SacI, PvuI und SalI begrenzt sind. Durch Doppelverdauung mit den entsprechenden Enzymen erhält man die Segmente (A) bis (F) oder auch zusammenhängende Segmente, beispielsweise mit EcoRI und MluI das Segment (A,B).

Beispiel B

Die Herstellung des Expressionsplasmids pEW1000 ist in der (nicht vorveröffentlichten) deutschen Patentanmeldung P 35 41 856.7 vorgeschlagen (Figur 1). Dieses Plasmid ist ein Derivat des Plasmids ptac 11 (Amann et al., Gene 25 (1983) 167 - 178), bei dem in die Erkennungsstelle für EcoRI eine synthetische Sequenz eingebaut wurde, die eine SalI-Schnittstelle enthält. Man erhält so das Expressionsplasmid pKK 177.3. Durch Insertion des lac-Repressors (Farabaugh, Nature 274 (1978) 765 - 769) erhält man das Plasmid pJF118. Dieses wird an der singulären Restriktionsschnittstelle für AvaI geöffnet und in bekannter Weise durch Exonuklease-Behandlung um etwa 1000 bp verkürzt und ligiert. Man erhält das Plasmid pEW1000. Durch Öffnen dieses Plasmids im Polylinker mit den Enzymen EcoRI und HindIII, SalI, PstI oder SmaI erhält man die linearisierten Expressionsplasmide (Ex1), (Ex2), (Ex3) und (Ex4).

Beispiel C

Das handelsübliche Plasmid pUC12 wird mit EcoRI und SalI geöffnet und das linearisierte Plasmid (1) isoliert. Durch Ligieren von (1) mit dem Segment (A), der synthetischen Linkersequenz (2) und dem Segment (F) erhält man das Plasmid pW226 (3).

Der Stamm E. coli 79/02 wird in bekannter Weise mit der Plasmid-DNA des Ligationsansatzes transformiert. Die Zellen werden auf Agarplatten ausplattiert, die Isopropyl-$\beta$-D-thiogalacto-pyranosid (IPTG), 5-Brom-4-chlor-3-indolyl-$\beta$-D-galactopyranosid (X-gal) sowie 20 $\mu$g/ml Ampicillin (Ap) enthalten. Von weißen Klonen wird die Plasmid-DNA gewonnen und durch Restriktionsanalyse und DNA-Sequenzanalyse die Bildung des Plasmids (3) bestätigt.

Aus dem Plasmid (3) wird das kleine EcoRI-HindIII-Fragment (4) ausgeschnitten und isoliert. Dieses wird in einer T4-DNA-Ligasereaktion mit dem linearisierten Expressionsplasmid (Ex1) ligiert. Das entstandene Plasmid pW226-1 (5) wird durch Restriktionsanalyse charakterisiert.

Kompetente Zellen des Stammes E. coli Mc 1061 werden mit DNA des Plasmides pW 226-1 transformiert. Klone, die resistent gegen Ampicillin sind, werden auf Ap-haltigen Agar-Platten isoliert. Die Plasmid-DNA wird aus Mc 1061-Zellen reisoliert und anschließend erneut mittels Restriktionsanalyse charakterisiert. Kompetente Zellen des E. coli-Stammes W 3110 werden nun mit Plasmid-DNA, die aus E. coli Mc 1061-Zellen isoliert wurde, transformiert. E. coli W 3110-Zellen werden im folgenden stets zur Expression benutzt. Entsprechend den folgenden Bedingungen werden alle Expressionsversuche zu den angegebenen Beispielen durchgeführt.

Eine Übernachtkultur aus E. coli-Zellen, die das Plasmid (5) enthalten, wird mit LB-Medium (J. H. Miller, Experiments in Molecular Genetics, Cold Spring Harbor Laboratory, 1972), das 50 $\mu$g/ml Ampicillin enthält, im Verhältnis von etwa 1:100 verdünnt und das Wachstum über OD-Messung verfolgt. Bei OD = 0,5 wird die Kultur auf 1 mM IPTG eingestellt und die Bakterien nach 150 bis 180 Minuten abzentrifugiert. Die Bakterien werden 5 Minuten in einer Puffermischung (7M Harnstoff, 0,1% SDS, 0,1 M Natriumphosphat, pH 7,0) gekocht und Proben auf eine SDS-Gelelektrophoreseplatte aufgetragen. Nach Elektrophorese wird aus Bakterien, die das Plasmid (5) enthalten, eine Proteinbande erhalten, die der Größe des erwarteten Proteins (6 kD) entspricht.

Die angegebenen Induktionsbedingungen gelten für Schüttelkulturen; bei größeren Fermentationen sind entsprechend veränderte OD-Werte und gegebenenfalls leicht variierte IPTG-Konzentrationen zweckmäßig.

Das erhaltene Protein zeigt keine biologische Aktivität im Zellproliferationstest mit einer IL-2-abhängigen Zellinie (CTLL 2).

Beispiel 1

Das Plasmid (3) wird mit MluI und SalI geöffnet und die beiden entstehenden Fragmente gelelektrophoretisch voneinander getrennt. Das größere Fragment (11) wird isoliert.

Das synthetische Oligonukleotid (12) wird mit der stumpfendigen, für Proinsulin kodierenden DNA (13) (Wetekam et al., Gene 19 (1982) 179 - 183) ligiert, wobei die DNA-Sequenz (14) erhalten wird. Diese wird mit MluI und SalI geschnitten, wobei die DNA-Sequenz (15) erhalten wird. Diese wird nun mit dem Fragment (11) ligiert, wobei das Plasmid pKH40 (16) gebildet wird. Dieses wird durch Restriktionsanalyse charakterisiert.

Das Plasmid (16) wird mit EcoRI und HindIII verdaut und das kleine Fragment (17) durch Gelelektrophorese isoliert. Durch Ligieren mit dem linearisierten Expressionsplasmid (Ex1) erhält man das Expressionsplasmid pK40 (18). Bei der Expression gemäß Beispiel C erhält man ein Protein, das nach Zellaufschluß in der löslichen zellulären Proteinfraktion gefunden wird. Die intakte Proinsulinsequenz wird durch "Western Blot"-Technik nachgewiesen.

Beispiel 2

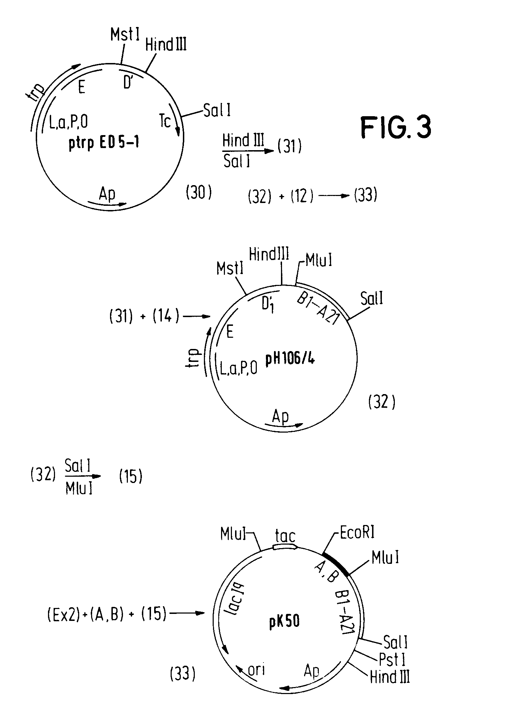

Ausgangsprodukt ist das Plasmid pPH30, das in der (nicht vorveröffentlichten) deutschen Patentanmeldung P 35 41 856.7 in Figur 3c dargestellt ist. Im Sinne der vorliegenden Erfindung wurde in Figur 2 die IL-2-Teilsequenz als "A-E" wiedergegeben (20). Das Ende dieser Sequenz und das Brückenglied bis zur Proinsulinsequenz ist in Figur 2 als (20a) wiedergegeben.

Das Plasmid (20) wird mit PvuI und HindIII verdaut und das kleine Fragment (22) isoliert. Weiterhin wird das Plasmid (3) mit EcoRI und PvuI geöffnet und das kleine Fragment (23) isoliert. Außerdem wird der Vektor pUC12 mit EcoRI und HindIII verdaut und das große Fragment (21) isoliert. Durch Ligieren der Fragmente (21), (23) und (22) erhält man das Plasmid pSL11 (24).

Das Plasmid (24) wird mit HindIII und partiell mit EcoRI verdaut und das Fragment (25) isoliert, das das Segment A und das Proinsulingen enthält. Durch Ligieren von (25) in das linearisierte Expressionsplasmid (Ex1) erhält man das Expressionsplasmid pSL12 (26).

Die Expression gemäß Beispiel C und die anschließende Aufarbeitung ergibt ein lösliches Fusionsprotein. Die "Western Blot"-Analyse mit Insulinantikörpern bestätigt, daß dieses Protein die intakte Insulinsequenz enthält.

Beispiel 3

Zur Amplifizierung des Proinsulingens dient das Plasmid ptrpED5-1 (30) (Hallewell et al., Gene 9 (1980) 27 - 47). Dieses wird mit HindIII und SalI geöffnet und das große Fragment (31) isoliert. Das Fragment (31) wird mit der DNA-Sequenz (14) ligiert, wobei das Plasmid pH106/4 (32) erhalten wird.

Das Plasmid (32) wird mit SalI und MluI verdaut und das kleine Fragment (15) isoliert. Das linearisierte Expressionsplasmid (Ex2), das Segment (A,B) und das Fragment (15) werden nun ligiert, wobei das Expressionsplasmid pK50 (33) erhalten wird.

Die Expression des kodierten Fusionsproteins wird gemäß Beispiel C durchgeführt. Die Zellen werden anschließend aus der Kulturbrühe abzentrifugiert und in der "French-Press" aufgebrochen. Die Proteinsuspension wird nun durch Zentrifugation in ihre löslichen und unlöslichen Proteinbestandteile separiert. Beide Fraktionen werden mittels Gelektrophorese in bekannter Weise auf 17,5 %igen SDS-Polyacrylamidgelen und anschließend durch Anfärbung der Proteine mit dem Farbstoff Coomassie Blue analysiert. Überraschend wird gefunden, daß das Fusionsprotein in dem unlöslichen Sediment gefunden wird. Die "Western Blot"-Analyse mit Insulinantikörpern bestätigt, daß intaktes Pro-Insulin im Fusionsprotein vorliegt.

Das Sediment des "French-Press"-Aufschlusses kann nun direkt zur Isolation von Pro-Insulin weiterverwendet werden.

Beispiel 4

Ausgangsprodukt ist das Plasmid pPH20 (40), das in der deutschen Patentanmeldung P 35 41 856.7 in Figur 3c dargestellt ist. Durch Schneiden dieses Plasmids mit EcoRI, Auffüllen der überstehenden Enden und Schneiden mit HindIII erhält man das Fragment (41), aus dem die DNA-Sequenz des hier interessierenden Teils von (40) ersichtlich ist.

Durch Ligieren des linearisierten Expressionsplasmids (Ex4) mit dem Segment (A,B), dem synthetischen Oligonukleotid (42) und dem Fragment (41) erhält man das Plasmid pK51 (43).

Beispiel 5

Durch Ligieren des linearisierten Expressionsplasmids (Ex2) mit dem Segment (A,B), dem synthetischen Oligonukleotid (51) und der DNA-Sequenz (15) erhält man das Plasmid pK52 (52). Die richtige Orientierung des Oligonukleotids (51) wird durch Sequenzanalyse entdeckt. Das Plasmid kodiert für ein Fusionsprotein, das die Aminosäuresequenz entsprechend dem Oligonukleotid (51) enthält und somit durch aktivierten Faktor Xa spaltbar ist.

Das Plasmid (52) ist auch auf dem folgenden Wege erhältlich:
Schneidet man das Plasmid (33) partiell mit MluI und ligiert das erhaltene geöffnete Plasmid (53) mit der DNA-Sequenz (51), so resultiert ebenfalls das Plasmid pK52.

Beispiel 6

Schneidet man das Plasmid (43) partiell mit MluI und ligiert das erhaltene linearisierte Plasmid (61) mit der synthetischen DNA-Sequenz (51), so erhält man das Plasmid pK53 (62). Dieses kodiert ebenfalls für ein Fusionsprotein, das mit dem aktivierten Faktor Xa spaltbar ist. Die richtige Orientierung der Sequenz (51) wird wie in Beispiel 5 durch DNA-Sequenzanalyse festgelegt.

Beispiel 7

Das Plasmid (26) wird mit XbaI und partiell mit MluI gespalten und das große Fragment (71) isoliert. Durch Ligieren mit dem Segment (c) erhält man das Plasmid pSL14 (72). Nach Expression und Zellaufschluß wird das Fusionsprotein in der löslichen zellulären Proteinfraktion gefunden.

Beispiel 8

Das Plasmid (20) wird mit XbaI und partiell mit EcoRI gespalten und die überstehenden Enden aufgefüllt, wobei die DNA-Sequenz (81) erhalten wird. Durch Ligieren unter "blunt end"-Bedingungen wird das Plasmid pPH31 (82) erhalten. Das Fusionsprotein wird in der unlöslichen zellulären Proteinfraktion gefunden.

Beispiel 9

Als Ausgangsmaterial dient das Plasmid (90), das in der EP-A 0 171 024 (Figur 3) beschrieben ist. Dieses Plasmid wird mit SalI und dann mit AccI umgesetzt und das kleine Fragment (91) isoliert. Dieses wird mit dem synthetischen Oligonukleotid (92) ligiert, wobei die DNA-Sequenz (93) erhalten wird. Diese wird mit MluI geschnitten, wobei das DNA-Fragment (94) resultiert.

Das Plasmid (33) wird mit MluI partiell und SalI verdaut und das große Fragment (95) isoliert. Dieses wird mit der DNA-Sequenz (94) ligiert, wobei das Expressionsplasmid pK192 (96) erhalten wird. Dieses kodiert für ein Fusionsprotein, bei dem auf die ersten 38 Aminosäuren von IL-2 Methionin und hierauf die Aminosäuresequenz von Hirudin folgen. Das Fusionsprotein wird in der löslichen zellulären Proteinfraktion gefunden.

Beispiel 10

Als Ausgangsmaterial dient das Plasmid pHG23 (100), das in der EP-A 0 183 350 beschrieben ist und das bei der American Type Culture Collection unter der Nr. ATCC 39000 allgemein zugänglich ist. Dieses Plasmid wird mit SfaNI geschnitten, die überstehenden Enden aufgefüllt, dann mit PstI umgesetzt und das kleine Fragment (101) isoliert. Durch Ligieren des linearisierten Expressionsplasmids (Ex3) mit dem Segment (A,B), dem synthetischen Oligonukleotid (102) und dem Fragment (101) erhält man das Expressionsplasmid pW214 (103). Dieses Plasmid kodiert für ein Fusionsprotein, in dem auf die ersten 38 Aminosäuren von IL-2 die Sequenz aus dem Oligonukleotid (102) folgt, welche die Spaltung des Moleküls mit Faktor Xa erlaubt, woran sich die Aminosäurefolge von CSF anschließt. Nach Zellaufschluß wird das Fusionsprotein in der unlöslichen zellulären Proteinfraktion gefunden.

Beispiel 11

Das Ausgangsplasmid pW216 (110) ist in der deutschen Patentanmeldung P 35 45 568.3 (Figur 2b) vorgeschlagen. In diesem Plasmid folgt auf die IL-2-Sequenz entsprechend den Segmenten A bis E (PvuI-Schnittstelle) ein Linker, der für die Aminosäuren Asp-Asp-Pro kodiert, unmittelbar gefolgt von der Aminosäurefolge für CSF. Die Verbindungssequenz zwischen IL-2 und CSF erlaubt die proteolytische Spaltung des Fusionsproteins.

Aus dem Plasmid (110) wird durch Schneiden mit PvuI und HindIII die Sequenz (111) isoliert.

Das Plasmid (3) wird MluI und XbaI geschnitten und das große Fragment (112) isoliert. Dieses wird mit dem Segment (C) ligiert, wobei das Plasmid pW227 (113) erhalten wird. Dieses Plasmid wird mit EcoRI und HindIII umgesetzt und das kurze Fragment (114) isoliert. Wird dieses Fragment mit dem linearisierten Expressionsplasmid (Ex1) ligiert, so erhält man das Plasmid pW227-1 (115). Das Plasmid kodiert für ein von IL-2 abgeleitetes Protein, das keine IL-2-Aktivität besitzt.

Das Plasmid (113) wird weiterhin mit EcoRI und PvuI geschnitten und das kurze Fragment (116) isoliert. Durch Ligieren des linearisierten Expressionsplasmids (Ex1) mit den Fragmenten (116) und (111) erhält man das Expressionsplasmid pW233 (117). Dieses kodiert für ein unlösliches Fusionsprotein, das auf Grund des vorstehend genannten Linkers proteolytisch spaltbar ist.

Beispiel 12

Das Plasmid (3) wird mit XbaI und SacI geschnitten und das große Fragment (121) isoliert. Durch Ligieren mit dem Segment (D) erhält man das Plasmid pW228 (122). Dieses wird mit EcoRI und HindIII geschnitten und das kleine Fragment (123) isoliert. Durch Ligieren des linearisierten Expressionsplasmids (Ex1) mit dem Fragment (123) erhält man das Expressionsplasmid pW228-1 (124). Dieses Plasmid kodiert ein biologich inaktives IL-2-Derivat. Dieses wird mit EcoRI und PvuI verdaut und das kurze Fragment (125) isoliert. Durch Ligieren des linearisierten Expressionsplasmids (Ex1) mit den Fragmenten (125) und (111) erhält man das Expressionsplasmid pW234 (126). Dieses kodiert für ein schwerlösliches Fusionsprotein, das ebenfalls proteolytisch spaltbar ist.

Beispiel 13

Zur Konstruktion von Plasmiden, die insbesondere für die Expression von cDNA-Sequenzen geeignet sind, wird zunächst die Polylinkersequenz (131) synthetisiert.

Durch Ligieren des linearisierten Plasmids (1) mit dem Segment (A), der Polylinkersequenz (131) und dem Segment (F) erhält man das Plasmid pH200 (132).

Das Plasmid (132) wird mit EcoRI und MluI umgesetzt und das große Fragment (133) isoliert. Wird dieses mit dem Segment (A,B) ligiert, so erhält man das Plasmid pH201 (134).

Das Plasmid (134) wird mit EcoRI und HindIII umgesetzt und das kurze Fragment (135) isoliert. Durch Ligieren dieses Fragments mit dem linearisierten Expressionsplasmid (Ex1) erhält man das Expressionsplasmid pH 202 (136).

Das Plasmid (136) wird mit BamHI geöffnet und in das linearisierte Plasmid die zu exprimierende cDNA über einen handelsüblichen BamHI-Adapter eingefügt. Je nach Orientierung der cDNA ist jede dritte Sequenz im Leseraster an (A,B) angeschlossen. Wenn die cDNA-Sequenz kein Stop-Codon enthält, so wird die von ihr kodierte Polypeptid-Sequenz zusätzlich durch die dem Segment (F) entsprechende Aminosäuresequenz geschützt.

Ist die cDNA nicht im richtigen Leseraster angeschlossen, so erzeugt man eine Verschiebung des Leserasters, indem man z.B. die cDNA-haltigen (ursprünglichen oder vermehrten) Plasmide mit MluI oder XbaI spaltet (sofern die cDNA keine Schnittstellen für diese Enzyme aufweist) und durch Klenow-Polymerasereaktion die überstehenden Enden auffüllt.

Anhang: DNA-Sequenz I

| Triplett Nr. | | | | | | | 0 | 1 | 2 |
|---|---|---|---|---|---|---|---|---|---|
| Aminosäure | | | | | | | Met | Ala | Pro |
| Nucleotid Nr. | | | | | 1 | | | 10 | |
| Cod. Strang | | | | 5' AA | TTC | ATG | GCG | CCG | |
| nicht cod. Strang | | | | 3' | G | | TAC | CGC | GGC |

(EcoRI)

| 3 | 4 | 5 | 6 | 7 | 8 | 9 | 10 | 11 | 12 |
|---|---|---|---|---|---|---|---|---|---|
| Thr | Ser | Ser | Ser | Thr | Lys | Lys | Thr | Gln | Leu |
| | 20 | | | | 30 | | | 40 | |
| ACC | TCT | TCT | TCT | ACC | AAA | AAG | ACT | CAA | CTG |
| TGG | AGA | AGA | AGA | TGG | TTT | TTC | TGA | GTT | GAC |

| 13 | 14 | 15 | 16 | 17 | 18 | 19 | 20 | 21 | 22 |
|---|---|---|---|---|---|---|---|---|---|
| Gln | Leu | Glu | His | Leu | Leu | Leu | Asp | Leu | Gln |
| | 50 | | | | 60 | | | 70 | |
| CAA | CTG | GAA | CAC | CTG | CTG | CTG | GAC | CTG | CAG |
| GTT | GAC | CTT | GTG | GAC | GAC | GAC | CTG | GAC | GTC |

PstI

| 23 | 24 | 25 | 26 | 27 | 28 | 29 | 30 | 31 | 32 |
|---|---|---|---|---|---|---|---|---|---|
| Met | Ile | Leu | Asn | Gly | Ile | Asn | Asn | Tyr | Lys |
| | 80 | | | | 90 | | | 100 | |
| ATG | ATC | CTG | AAC | GGT | ATC | AAC | AAC | TAC | AAA |
| TAC | TAG | GAC | TTG | CCA | TAG | TTG | TTG | ATG | TTT |

| 33 | 34 | 35 | 36 | 37 | 38 | 39 | 40 | 41 | 42 |
|---|---|---|---|---|---|---|---|---|---|
| Asn | Pro | Lys | Leu | Thr | Arg | Met | Leu | Thr | Phe |
| | 110 | | | | 120 | | | 130 | |
| AAC | CCG | AAA | CTG | ACG | CGT | ATG | CTG | ACC | TTC |
| TTG | GGC | TTT | GAC | TGC | GCA | TAC | GAC | TGG | AAG |

MluI

9

| 43 | 44 | 45 | 46 | 47 | 48 | 49 | 50 | 51 | 52 |
|----|----|----|----|----|----|----|----|----|----|
| Lys | Phe | Tyr | Met | Pro | Lys | Lys | Ala | Thr | Glu |
|  | 140 |  |  |  | 150 |  |  | 160 |  |
| AAA | TTC | TAC | ATG | CCG | AAA | AAA | GCT | ACC | GAA |
| TTT | AAG | ATG | TAC | GGC | TTT | TTT | CGA | TGG | CTT |

| 53 | 54 | 55 | 56 | 57 | 58 | 59 | 60 | 61 | 62 |
|----|----|----|----|----|----|----|----|----|----|
| Leu | Lys | His | Leu | Gln | Cys | Leu | Glu | Glu | Glu |
|  | 170 |  |  |  | 180 | _____ |  | 190 |  |
| CTG | AAA | CAC | CTC | CAG | TGT | CTA | GAA | GAA | GAG |
| GAC | TTT | GTG | GAG | GTC | ACA | GAT | CTT | CTT | CTC |

XbaI

| 63 | 64 | 65 | 66 | 67 | 68 | 69 | 70 | 71 | 72 |
|----|----|----|----|----|----|----|----|----|----|
| Leu | Lys | Pro | Leu | Glu | Glu | Val | Leu | Asn | Leu |
|  | 200 |  |  |  | 210 |  |  | 220 |  |
| CTG | AAA | CCG | CTG | GAG | GAA | GTT | CTG | AAC | CTG |
| GAC | TTT | GGC | GAC | CTC | CTT | CAA | GAC | TTG | GAC |

| 73 | 74 | 75 | 76 | 77 | 78 | 79 | 80 | 81 | 82 |
|----|----|----|----|----|----|----|----|----|----|
| Ala | Gln | Ser | Lys | Asn | Phe | His | Leu | Arg | Pro |
|  | 230 |  |  |  | 240 |  |  | 250 |  |
| GCT | CAG | TCT | AAA | AAT | TTC | CAC | CTG | CGT | CCG |
| CGA | GTC | AGA | TTT | TTA | AAG | GTG | GAC | GCA | GGC |

| 83 | 84 | 85 | 86 | 87 | 88 | 89 | 90 | 91 | 92 |
|----|----|----|----|----|----|----|----|----|----|
| Arg | Asp | Leu | Ile | Ser | Asn | Ile | Asn | Val | Ile |
|  | 260 |  |  |  | 270 |  |  | 280 |  |
| CGT | GAC | CTG | ATC | TCT | AAC | ATC | AAC | GTT | ATC |
| GCA | CTG | GAC | TAG | AGA | TTG | TAG | TTG | CAA | TAG |

| 93 | 94 | 95 | 96 | 97 | 98 | 99 | 100 | 101 | 102 |
|----|----|----|----|----|----|----|----|----|----|
| Val | Leu | Glu | Leu | Lys | Gly | Ser | Glu | Thr | Thr |
|  | 290 | _____ |  |  | 300 |  |  | 310 |  |
| GTT | CTG | GAG | CTC | AAA | GGT | TCT | GAA | ACC | ACG |
| CAA | GAC | CTC | GAG | TTT | CCA | AGA | CTT | TGG | TGC |

SacI

| 103 | 104 | 105 | 106 | 107 | 108 | 109 | 110 | 111 | 112 |
|-----|-----|-----|-----|-----|-----|-----|-----|-----|-----|
| Phe | Met | Cys | Glu | Tyr | Ala | Asp | Glu | Thr | Ala |
|     | 320 |     |     |     | 330 |     |     | 340 |     |
| TTC | ATG | TGC | GAA | TAC | GCG | GAC | GAA | ACT | GCG |
| AAG | TAC | ACG | CTT | ATG | CGC | CTG | CTT | TGA | CGC |

| 113 | 114 | 115 | 116 | 117 | 118 | 119 | 120 | 121 | 122 |
|-----|-----|-----|-----|-----|-----|-----|-----|-----|-----|
| Thr | Ile | Val | Glu | Phe | Leu | Asn | Arg | Trp | Ile |
|     | 350 |     |     |     | 360 |     |     | 370 |     |
| ACG | ATC | GTT | GAA | TTT | CTG | AAC | CGT | TGG | ATC |
| TGC | TAG | CAA | CTT | AAA | GAC | TTG | GCA | ACC | TAG |
|     | PvuI |    |     |     |     |     |     |     |     |

| 123 | 124 | 125 | 126 | 127 | 128 | 129 | 130 | 131 | 132 |
|-----|-----|-----|-----|-----|-----|-----|-----|-----|-----|
| Thr | Phe | Cys | Gln | Ser | Ile | Ile | Ser | Thr | Leu |
|     | 380 |     |     |     | 390 |     |     | 400 |     |
| ACC | TTC | TGC | CAG | TCG | ATC | ATC | TCT | ACC | CTG |
| TGG | AAG | ACG | GTC | AGC | TAG | TAG | AGA | TGG | GAC |

| 133 | 134 | 135 |     |   |     |
|-----|-----|-----|-----|---|-----|
| Thr |     |     |     |   |     |
|     | 410 |     |     |   |     |
| ACC | TGA | TAG |     |   | 3' |
| TGG | ACT | ATC | AGC | T | 5' |
|     |     | (SalI) |  |   |     |

## Patentansprüche

**Patentansprüche für folgende Vertragsstaaten : BE, CH, DE, FR, GB, GR, IT, LI, LU, NL, SE**

1. Fusionsproteine mit einem Anteil von Interleukin-2 (IL-2) und einem gewünschten Protein, gekennzeichnet durch einen C- oder N-terminalen Anteil, der im wesentlichen der Aminosäurefolge von IL-2 entspricht, der aber biologisch nicht aktiv ist, wobei Fusionsproteine ausgenommen sind, deren IL-2-Anteil im wesentlichen mindestens den ersten 100 Aminosäuren von IL-2 entspricht.

2. Fusionsproteine nach Anspruch 1, dadurch gekennzeichnet, daß die Aminosäurefolge der des menschlichen IL-2 entspricht.

3. Fusionsproteine nach Anspruch 2, dadurch gekennzeichnet, daß das für IL-2 kodierende Gen einen Teil der DNA-Sequenz I (Anhang) umfaßt.

4. Fusionsprotein nach Anspruch 3, dadurch gekennzeichnet, daß das für den IL-2-Anteil kodierende Gen im wesentlichen aus einem, zwei oder drei der Segmente A bis F des IL-2-Gens

   (EcoRI)-A-PstI-B-MluI-C-XbaI-D-SacI-E-PvuI-F-(SalI)

   in beliebiger Reihenfolge, gegebenenfalls über Adapter- oder Linkersequenzen verknüpft, besteht.

5. Fusionsproteine nach einem oder mehreren der vorhergehenden Ansprüche, dadurch gekennzeichnet,

11

daß zwischen der IL-2-Sequenz und der Aminosäuresequenz des gewünschten Proteins eine Aminosäure oder Aminosäurefolge angeordnet ist, die die chemische oder enzymatische Abspaltung des gewünschten Proteins vom IL-2-Anteil ermöglicht.

6. Fusionsprotein nach Anspruch 5, dadurch gekennzeichnet, daß die Aminosäure Met, Cys, Trp, Lys oder Arg ist oder die Aminosäurefolge diese Aminosäuren C-terminal enthält.

7. Fusionsprotein nach Anspruch 6, dadurch gekennzeichnet, daß die Aminosäurefolge Asp-Pro ist oder C-terminal diese Aminosäurefolge enthält.

8. Fusionsprotein nach Anspruch 6, dadurch gekennzeichnet, daß die Aminosäurefolge Ile-Glu-Gly-Arg ist oder C-terminal diese Aminosäurefolge enthält.

9. Verfahren zur Herstellung der Fusionsproteine nach einem oder mehreren der Ansprüche 1 bis 8, dadurch gekennzeichnet, daß ein für das Fusionsprotein kodierendes Gen in einer Wirtszelle zur Expression gebracht wird.

10. Verfahren nach Anspruch 9, dadurch gekennzeichnet, daß das Gen in einen Expressionsvektor eingebaut und in einer Bakterienzelle exprimiert wird.

11. Verfahren nach Anspruch 10, dadurch gekennzeichnet, daß als Bakterienzelle E. coli dient.

12. Verwendung der Fusionsproteine nach Anspruch 1 bis 8 bzw. der nach Anspruch 9 bis 11 erhältlichen Fusionsproteine zur Herstellung der gewünschten Proteine.

13. Genstruktur, kodierend für ein Fusionsprotein nach Anspruch 1 bis 8.

14. Vektor, enthaltend eine Genstruktur nach Anspruch 13.

15. pUC12-Derivate pW226 (Fig. C), pW227 (Fig. 11), pW228 (Fig. 12), pH 200 und pH 201 (Fig. 13); Expressionsplasmide pW226-1 (Fig. C), pW227-1 (Fig. 11), pW228-1 (Fig. 12) und pH 202 (Fig. 13); Expressionsplasmide, codierend für ein Fusionsprotein aus einem Il-2-Anteil gemäß Anspruch 1, einem Brückenglied und Proinsulin: pK40 (Fig. 1), pSL12 (Fig. 2), pK50 (Fig. 3), pK51 (Fig. 4), pK52 (Fig. 5), pK53 (Fig. 6), pSL14 (Fig. 7), pPH31 (Fig. 8); Expressionsplasmid, codierend für ein Fusionsprotein aus einem IL-2-Anteil gemäß Anspruch 1, einem Brückenglied und Hirudin: pK 192 (Fig. 9); Expressionsplasmide, codierend für ein Fusionsprotein aus einem IL-2-Anteil gemäß Anspruch 1, einem Brückenglied und GM-CSF: pW214 (Fig. 10), pW233 (Fig. 11), pW234 (Fig. 12).

16. Wirtszelle, enthaltend einen Vektor nach Anspruch 14 oder 15.

**Patentansprüche für folgende Vertragsstaaten : AT, ES**

1. Verfahren zur Herstellung eines Fusionsproteins mit einem Anteil von Interleukin-2 (IL-2) und einem gewünschten Protein mit einem C- oder N-terminalen Anteil, der im wesentlichen der Aminosäurefolge von IL-2 entspricht, der aber biologisch nicht aktiv ist, dadurch gekennzeichnet, daß ein für das Fusionsprotein codierendes Gen in eine Wirtszelle zur Expression gebracht wird, wobei die Herstellung von Fusionsproteinen ausgenommen ist, deren IL-2-Anteil im wesentlichen mindestens den ersten 100 Aminosäuren von IL-2 entspricht.

2. Verfahren nach Anspruch 1, dadurch gekennzeichnet, daß die Aminosäurefolge des IL-2-Anteils der des menschlichen IL-2 entspricht.

3. Verfahren nach Anspruch 2, dadurch gekennzeichnet, daß das für IL-2 kodierende Gen einen Teil der DNA-Sequenz I (Anhang) umfaßt.

4. Verfahren nach Anspruch 3, dadurch gekennzeichnet, daß das für den IL-2-Anteil kodierende Gen im wesentlichen aus einem, zwei oder drei der Segmente A bis F des IL-2-Gens

(EcoRI)-A-PstI-B-MluI-C-XbaI-D-SacI-E-PvuI-F-(SalI)

in beliebiger Reihenfolge, gegebenenfalls über Adapter- oder Linkersequenzen verknüpft, besteht.

5. Verfahren nach einem oder mehreren der vorhergehenden Ansprüche, dadurch gekennzeichnet, daß zwischen der IL-2-Sequenz und der Aminosäuresequenz des gewünschten Proteins eine Aminosäure oder Aminosäurefolge angeordnet ist, die die chemische oder enzymatische Abspaltung des gewünschten Proteins vom IL-2-Anteil ermöglicht.

6. Verfahren nach Anspruch 5, dadurch gekennzeichnet, daß die Aminosäure Met, Cys, Trp, Lys oder Arg ist oder die Aminosäurefolge diese Aminosäuren C-terminal enthält.

7. Verfahren nach Anspruch 6, dadurch gekennzeichnet, daß die Aminosäurefolge Asp-Pro ist oder C-terminal diese Aminosäurefolge enthält.

8. Verfahren nach Anspruch 6, dadurch gekennzeichnet, daß die Aminosäurefolge Ile-Glu-Gly-Arg ist oder C-terminal diese Aminosäurefolge enthält.

9. Verfahren einem oder mehreren der vorhergehenden Ansprüche, dadurch gekennzeichnet, daß das Gen in einen Expressionsvektor eingebaut und in einer Bakterienzelle, vorzugsweise E. coli, exprimiert wird.

10. Verwendung der nach Anspruch 1 bis 9 erhältlichen Fusionsproteine zur Herstellung der gewünschten Proteine.

## Claims
## Claims for the following Contracting States : BE, CH, DE, FR, GB, GR, IT, LI, LU, NL, SE

1. A fusion protein having an interleukin-2 (IL-2) portion and a desired protein, which has the feature that it contains a C- or N-terminal portion which essentially corresponds to the amino acid sequence of IL-2 but which is not biologically active, excepting fusion proteins whose IL-2 portion corresponds essentially at least to the first 100 amino acids of IL-2.

2. A fusion protein as claimed in claim 1, wherein the amino acid sequence corresponds to that of human IL-2.

3. A fusion protein as claimed in claim 2, wherein the gene coding for IL-2 contains part of DNA sequence I (addendum).

4. A fusion protein as claimed in claim 3, wherein the gene coding for the IL-2 portion is essentially composed of one, two or three of the segments A to F of the IL-2 gene

(EcoRI)-A-PstI-B-MluI-C-XbaI-D-SacI-E-PvuI-F-(SalI)

in arbitrary sequence, where appropriate linked via adaptor or linker sequences.

5. A fusion protein as claimed in one or more of the preceding claims, wherein is located, between the IL-2 sequence and the amino acid sequence of the desired protein, an amino acid or amino acid sequence which allows the desired protein to be cleaved off, chemically or enzymatically, from the IL-2 portion.

6. A fusion protein as claimed in claim 5, wherein the amino acid is Met, Cys, Trp, Lys or Arg, or the amino acid sequence contains these amino acids at the C-terminal end.

7. A fusion protein as claimed in claim 6, wherein the amino acid sequence is Asp-Pro or contains this amino acid sequence at the C-terminal end.

8. A fusion protein as claimed in claim 6, wherein the amino acid sequence is Ile-Glu-Gly-Arg or contains this amino acid sequence at the C-terminal end.

9.  A process for the preparation of the fusion proteins as claimed in one or more of claims 1 to 8, which comprises causing the expression of a gene coding for the fusion protein in a host cell.

10. The process as claimed in claim 9, wherein the gene is incorporated in an expression vector and is expressed in a bacterial cell.

11. The process as claimed in claim 10, wherein the bacterial cell used is E. coli.

12. The use of the fusion proteins as claimed in claims 1 to 8, or of the fusion proteins obtainable as claimed in claims 9 to 11, for the preparation of the desired proteins.

13. A gene structure coding for a fusion protein as claimed in claims 1 to 8.

14. A vector containing a gene structure as claimed in claim 13.

15. The pUC12 derivatives pW226 (Fig. C), pW227 (Fig. 11), pW228 (Fig. 12), pH 200 and pH 201 (Fig. 13); expression plasmids pW226-1 (Fig. C), pW227-1 (Fig. 11), pW228-1 (Fig. 12) and pH 202 (Fig. 13); expression plasmids coding for a fusion protein composed of an IL-2 portion as claimed in claim 1, of a bridging element and proinsulin: pK40 (Fig. 1), pSL12 (Fig. 2), pK50 (Fig. 3), pK51 (Fig. 4), pK52 (Fig. 5), pK53 (Fig. 6), pSL14 (Fig. 7), pPH31 (Fig. 8); expression plasmid coding for a fusion protein composed of an IL-2 portion as claimed in claim 1, of a bridging element and hirudin: pK192 (Fig. 9); expression plasmids coding for a fusion protein composed of an IL-2 portion as claimed in claim 1, of a bridging element and GM-CSF: pW214 (Fig. 10), pW233 (Fig. 11), pW234 (Fig. 12).

16. A host cell containing a vector as claimed in claim 14 or 15.

**Claims for the following Contracting States : AT, ES**

1.  A process for the preparation of a fusion protein having an interleukin-2 (IL-2) portion and a desired protein having a C- or N-terminal portion which essentially corresponds to the amino acid sequence of IL-2 but which is not biologically active, which comprises causing the expression of a gene coding for the fusion protein in a host cell, excepting the preparation of fusion proteins whose IL-2 portion corresponds essentially at least to the first 100 amino acids of IL-2.

2.  The process as claimed in claim 1, wherein the amino acid sequence of the IL-2 portion corresponds to that of human IL-2.

3.  The process as claimed in claim 2, wherein the gene coding for IL-2 Contains part of DNA sequence I (addendum).

4.  The process as claimed in claim 3, wherein the gene coding for the IL-2 portion is essentially composed of one, two or three of the segments A to F of the IL-2 gene

    (EcoRI)-A-PstI-B-MluI-C-XbaI-D-SacI-E-PvuI-F-(SalI)

    in arbitrary sequence, where appropriate linked via adaptor or linker sequences.

5.  The process as claimed in one or more of the preceding claims, wherein is located, between the IL-2 sequence and the amino acid sequence of the desired protein, an amino acid or amino acid sequence which allows the desired protein to be cleaved off, chemically or enzymatically, from the IL-2 portion.

6.  The process as claimed in claim 5, wherein the amino acid is Met, Cys, Trp, Lys or Arg, or the amino acid sequence contains these amino acids at the C-terminal end.

7.  The process as claimed in claim 6, wherein the amino acid sequence is Asp-Pro or contains this amino acid sequence at the C-terminal end.

**8.** The process as claimed in claim 6, wherein the amino acid sequence is Ile-Glu-Gly-Arg or contains this amino acid sequence at the C-terminal end.

**9.** The process as claimed in one or more of the preceding claims, wherein the gene is incorporated in an expression vector and is expressed in a bacterial cell, preferably E. coli.

**10.** The use of the fusion proteins obtainable as claimed in claims 1 to 9, for the preparation of the desired proteins.

**Revendications**
**Revendications pour les Etats contractants suivants : BE, CH, DE, FR, GB, GR, IT, LI, LU, NL, SE**

**1.** Protéines de fusion comportant une fraction d'interleukine-2 (IL-2) et une protéine recherchée, caractérisée par une fraction C-terminale ou N-terminale qui correspond principalement à la séquence d'amino-acides de l'interleukine-2 mais qui n'est pas biologiquement active, à l'exclusion des protéines de fusion dont la fraction d'IL-2 correspond, principalement, au moins aux 100 premiers amino-acides de l'IL-2.

**2.** Protéines de fusion selon la revendication 1, caractérisées en ce que la séquence d'amino-acides correspond à celle de l'IL-2 humaine.

**3.** Protéines de fusion selon la revendication 2, caractérisées en ce que le gène codant pour l'IL-2 comprend une partie de la séquence d'ADN I (annexe).

**4.** Protéines de fusion selon la revendication 3, caractérisées en ce que le gène codant pour la fraction d'IL-2 se compose d'un, de deux ou de trois des segments A à F du gène de l'IL-2

(EcoRI)-A-PstI-B-MluI-C-XbaI-D-SacI-E-PvuI-F-(SalI),

disposé(s) dans un ordre quelconque, éventuellement lié(s) à des séquences d'adaptateurs ou de linkers.

**5.** Protéines de fusion selon une ou plusieurs des revendications précédentes, caractérisées en ce que, entre la séquence d'IL-2 et la séquence d'amino-acides de la protéine recherchée, on place un amino-acide ou une séquence d'amino-acides qui permet de séparer la protéine recherchée de la fraction d'IL-2, par voie chimique ou enzymatique.

**6.** Protéine de fusion selon la revendication 5, caractérisée en ce que l'amino-acide est Met, Cys, Trp, Lys ou Arg, ou la séquence d'amino-acides contient ces amino-acides en position C-terminale.

**7.** Protéine de fusion selon la revendication 6, caractérisée en ce que la séquence d'amino-acides est Asp-Pro ou contient cette séquence d'amino-acides en position C-terminale.

**8.** Protéine de fusion selon la revendication 6, caractérisée en ce que la séquence d'amino-acides est Ile-Glu-Gly-Arg ou contient cette séquence d'amino-acides en position C-terminale.

**9.** Procédé pour préparer des protéines de fusion selon une ou plusieurs des revendications 1 à 8, caractérisé en ce que l'on apporte un gène d'expression codant pour la protéine de fusion dans une cellule-hôte.

**10.** Procédé selon la revendication 9, caractérisé en ce qu'on introduit le gène dans un vecteur d'expression et on l'exprime dans une cellule bactérienne.

**11.** Procédé selon la revendication 10, caractérisé en ce que E. coli sert de cellule bactérienne.

**12.** Utilisation des protéines de fusion selon les revendications 1 à 8, ou protéines de fusion obtenues selon les revendications 9 à 11, pour Préparer les protéines recherchées.

**13.** Structure génique codant pour une protéine de fusion selon les revendications 1 à 8.

**14.** Vecteur contenant une structure génique selon la revendication 13.

**15.** Dérivés de pUC12: pW226 (figure C), pW227 (figure 11), pW228 (figure 12), pH 200. et pH 201 (figure 13); plasmides d'expression pW226-1 (figure C), pW227-1 (figure 11), pW228-1 (figure 12) et pH 202 (figure 13); plasmides d'expression codant pour une protéine de fusion entre une fraction d'IL-2 selon la revendication 1, un élément de pont et la proinsuline: pK40 (figure 1), pSL12 (figure 2), pK50 (figure 3), pK51 (figure 4), pK52 (figure 5), pK53 (figure 6), pSL14 (figure 7), pPH31 (figure 8); plasmide d'expression codant pour une protéine de fusion entre une fraction d'IL-2 selon la revendication 1, un élément de pont et l'hirudine: pK 192 (figure 9); plasmides d'expression codant pour une protéine de fusion entre une fraction d'IL-2 selon la revendication 1, un élément de pont et le GM-CSF: pW214 (figure 10), pW233 (figure 11), pW234 (figure 12).

**16.** Cellule-hôte contenant un vecteur selon la revendication 14 ou 15.

**Revendications pour les Etats contractants suivants : AT, ES**

**1.** Procédé pour préparer une protéine de fusion comportant une fraction d'interleukine-2 (IL-2) et une protéine recherchée ayant une fraction C-terminale ou N-terminale qui correspond principalement à la séquence d'amino-acides de l'interleukine-2 mais qui n'est pas biologiquement active, caractérisé en ce qu'on apporte un gène d'expression codant pour la protéine de fusion dans une cellule-hôte, la préparation de protéines de fusion dont la fraction d'IL-2 correspond, principalement, au moins aux 100 premiers amino-acides de l'IL-2, étant exclue.

**2.** Procédé selon la revendication 1, caractérisé en ce que la séquence d'amino-acides de la fraction d'IL-2 correspond à celle de l'IL-2 humaine.

**3.** Procédé selon la revendication 2, caractérisé en ce que le gène codant pour l'IL-2 comprend une partie de la séquence d'ADN I (annexe).

**4.** Procédé selon la revendication 3, caractérisé en ce que le gène codant pour la fraction d'IL-2 se compose pricipalement d'un, de deux ou de trois des segments A à F du gène de l'IL-2

(EcoRI)-A-PstI-B-MluI-C-XbaI-D-SacI-E-PvuI-F-(SalI),

disposé(s) dans un ordre quelconque, éventuellement lié(s) à des séquences d'adaptateurs ou de linkers.

**5.** Procédé selon une ou plusieurs des revendications précédentes, caractérisé en ce que, entre la séquence d'IL-2 et la séquence d'amino-acides de la protéine recherchée, on place un amino-acide ou une séquence d'amino-acides qui permet de séparer la protéine recherchée de la fraction d'IL-2, par voie chimique ou enzymatique.

**6.** Procédé selon la revendication 5, caractérisé en ce que l'amino-acide est Met, Cys, Trp, Lys ou Arg, ou la séquence d'amino-acides contient ces amino-acides en position C-terminale.

**7.** Procédé selon la revendication 6, caractérisé en ce que la séquence d'amino-acides est Asp-Pro ou contient cette séquence d'amino-acides en position C-terminale.

**8.** Procédé selon la revendication 6, caractérisé en ce que la séquence d'amino-acides est Ile-Glu-Gly-Arg ou contient cette séquence d'amino-acides en position C-terminale.

**9.** Procédé selon une ou plusieurs des revendications précédentes, caractérisé en ce qu'on introduit le gène dans un vecteur d'expression et on l'exprime dans une cellule bactérienne, de préférence dans E. coli.

**10.** Utilisation des protéines de fusion obtenues selon les revendications 1 à 9 pour préparer les protéines

recherchées.

# FIG. A

$$\frac{EcoRI}{PstI} \rightarrow (EcoRI)-A-(PstI) \quad (A)$$

$$\frac{PstI}{MluI} \rightarrow (PstI)-B-(MluI) \quad (B)$$

$$\frac{MluI}{XbaI} \rightarrow (MluI)-C-(XbaI) \quad (C)$$

$$\frac{XbaI}{SacI} \rightarrow (XbaI)-D-(SacI) \quad (D)$$

$$\frac{SacI}{PvuI} \rightarrow (SacI)-E-(PvuI) \quad (E)$$

$$\frac{PvuI}{SalI} \rightarrow (PvuI)-F-(SalI) \quad (F)$$

$$\frac{EcoRI}{MluI} \rightarrow (EcoRI)-A-Pst-B-(MluI) \quad (A,B)$$

# FIG. B

$$\frac{EcoRI}{HindIII} \rightarrow (Ex1)$$

$$\frac{EcoRI}{SalI} \rightarrow (Ex2)$$

$$\frac{EcoRI}{PstI} \rightarrow (Ex3)$$

$$\frac{EcoRI}{SmaI} \rightarrow (Ex4)$$

# FIG. C

pUC 12 $\xrightarrow{\text{EcoRI}}$ 
          $\text{SalI}$

```
        G              TCGAC
      C TTAA             G
      (EcoRI)          (SalI)
```
(1)

```
       MluI    XbaI        SacI
    G ACG CGT CTA GAA  GAG CTC ACG AT
   AC GTC TGC GCA GAT CTT  CTC GAG TGC
   (PstI)                        (PvuI)
```
(2)

(1) + (A) + (2) + (F) $\longrightarrow$ PstI

pW226 (pUC12)

(3)

(3) $\xrightarrow[\text{HindIII}]{\text{EcoRI}}$ (EcoRI)-**A**-PstI-MluI-XbaI-SacI-PvuI-**F**-SalI-PstI-(HindIII)

(4)

(Ex1) + (4) $\longrightarrow$

pW226-1

# FIG.1

$(3) \dfrac{MluI}{SalI} \rightarrow$

| A | CTG CAG A | | TCG AC |
|---|---|---|---|
| | GAC GTC TGC GC | | G |
| | | (MluI) | (SalI) | (11)

MluI
B1
Phe
A21
Asn
SalI

AGCTTCCATG ACG CGT    TTT    (B2-A20)    AAC TAA TAG TCGAC
       AGGTAC TGC GCA    AAA              TTG ATT ATC AGCTG
(HindIII)
         (12)                              (13)

(12) + (13) ⟶ (14)

$(14) \dfrac{MluI}{SalI} \rightarrow$    CGCGT TTT    (B2-A20)    AAC TAA TAG    (15)
                        (MluI) A AAA                 TTG ATT ATC AGC T
                                                              SalI

(11) + (15) ⟶

pKH40
pUC12
(16)

PstI · SalI · HindIII · MluI · PstI · A · EcoRI · B1-A21

$(16) \dfrac{EcoRI}{HindIII} \rightarrow$ (EcoRI)-A-MluI-(B1-A21)-SalI-(HindIII)  (17)

(Ex1) + (17) ⟶

pK40
tac · EcoRI · PstI · MluI · B1-A21 · SalI · PstI · HindIII · Ap · ori · lacIq
(18)

EP 0 229 998 B1

FIG.2

113 114 EcoRI
Thr Ile Asp Phe Met Ile Thr Thr Tyr Ser Leu Ala Ala Gly Arg (20a)
ACG ATC GAA TTC ATG ATC ACA ACG TAT AGC TTG GCT GCA GGT CGC
TGC TAG CTT AAG TAC TAG TGT TGC ATA TCG AAC CGA CGT CCA GCG
Pvu I

$(20) \dfrac{PvuI}{HindIII}$ (PvuI)–EcoRI ≠ B1–A21–SalI–Pst–(HindIII) (22)

$(3) \dfrac{EcoRI}{PvuI}$ (EcoRI)–A–PstI–MluI–XbaI–SacI–(PvuI) (23)

(21) + (23) + (22) ⟶

$(24) \dfrac{HindIII}{EcoRI, part.}$

(EcoRI)–A–PstI # EcoRI #
–B1–A21–SalI–PstI–(HindIII)
(25)

(Ex1) + (25) ⟶

21

FIG. 3

FIG.4

(Ser) (Leu) Ala Ala Gly Arg          Stp  Stp
 AGC  TTG  GCT GCA GGT CGC (B1-A21) TAA TAG TCG AGG GAA TT
      AC  CGA CGT CCA GCG            ATT ATC AGC TCC CTT AA  (41)
(Hind III)                                        (EcoRI⁻)

              38
          (Arg) Leu Gln Met Pro (Ser)
          CG CGT CTG CAG ATG CCA              (42)
             A GAC GTC TAC GGT TCG A
       (MluI)                    (Hind III)

(Ex4) + (A,B)+(42) + (41) →

23

```
(Arg) Ile  Glu  Gly  Arg(Thr)
CG CGT ATC GAA GGT CGT A
    A TAG C T T  CCA GCA TGC GC          (51)
(MluI)                      (MluI)
```

**FIG.5**

pK52

(Ex2)+ (A,B) + (51) + (15) ———▶

(52)

```
(33) ──Mlu I. part.──▶
(53) + (51) ──▶ (52)
```

**FIG.6**

```
(43) ──MluI──▶ (61)
(61) + (51) ──▶
```

pK53

(62)

24

# FIG.7

# FIG.8

FIG.9

Sal1 (Acc1)
Sst1 (Sac1)
Hirudin
Ap
Acc1
Xbal
pUC 12
ori
(90)

$\dfrac{\text{Sal I}}{\text{Acc I}}$ →

3
Thr                    Stp Stp
AT ACT                TAA TAG AGC TCG          (91)
   TGA (Hir4-64) ATT ATC TCG AGC AGC T
(Acc I)                                    (Sal I)

                0  1   2
      Mlu I     Met Thr (Tyr)
CCC ACG CGT ATG ACG T          (92)
GGG TCC GCA TAC TGC ATA
                    (Acc I)
(91)+(92) ⟶ (93) $\xrightarrow{\text{Mlu I}}$ (94)

(33) $\xrightarrow[\text{Sal I}]{\text{Mlu I, part.}}$  Mlu I   tac   EcoRI   (Mlu I)
                                          A , B                    (95)
                    Hind III        (Sal I)
                         Pst I

Mlu I   tac        EcoRI
                        A,B   Mlu I
(94)+(95) ⟶  lacIq  pK 192  Hirudin
                                    Sal I
                                    Pst I
(96)        ori    Ap    Hind III

# FIG.10

FIG.11

FIG.12

FIG.13